# EUROPEAN PATENT APPLICATION

(11) **EP 4 199 159 A1**
(43) Date of publication of application: **21.06.2023**
(21) Application number: 21306821.6
(22) Date of filing: 17.12.2021
(51) Int. Cl.: H01M 4/86, H01M 4/90, H01M 8/16

(54) **REDOX MEDIATOR ENCAPSULATION**

(71) Applicant: BeFC, 38000 Grenoble (FR)
(72) Inventor: SOLAN, Sébastien, 38400 SAINT MARTIN D'HERES (FR); CESBRON, Marius, 87360 AZAT LE RIS (FR); BESSAC, Elise, 38000 GRENOBLE (FR); TORASSO, William, 38430 MOIRANS (FR); BLOCH, Jean-Francis, 38610 GIERES (FR); HAMMOND, Jules, 38610 GIERES (FR)
(74) Representative: Novagraaf Technologies

(57) **Abstract**

The present invention concerns a method for the encapsulation of a redox mediator, and the use of such encapsulated redox mediator as electrode materials.

## Description

### Technical field of the invention

The present invention relates to a method for the encapsulation of a redox mediator, and the use of such encapsulated redox mediator as electrode materials.

In the description below, references in square brackets ([ ]) refer to the list of references at the end of the text.

### State of the art

Biological fuel cells (Biofuel cells) offer an attractive means to provide eco-friendly and sustainable power to electronic devices, particularly for small portable devices for applications such as healthcare, environmental monitoring, bio-defense, etc. Given that enzyme-based fuel cells can operate using substrates that are abundant in the biological fluids and environmental effluent (*i.e.,* glucose and oxygen) whilst exhibiting power densities that are often superior to microbial fuel cells, they offer an attractive proposition to augment or self-power miniaturized wearable or implantable devices [1,2,3]. Furthermore, paper-based devices are gaining popularity as propositions for these types of applications owing to their low mass, small form factor and flexibility, allowing them to conform to a range of different surfaces.

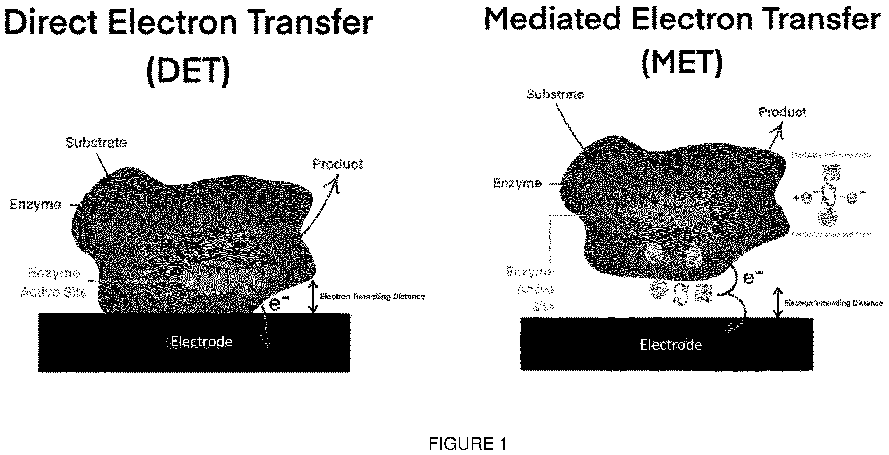

The core of this technology is based on a couple a of redox enzymes that ensure the catalysis of the fuels. Redox enzymes have a protein structure (apoenzyme) comprising an active redox cofactor capable of transferring electrons from the substrate to the co-substrate. In the case where the redox protein is capable of exchanging these electrons with the electrode, then replacing the substrate or co-substrate, this is called a direct electronic transfer (DET) process (Figure 1).

This phenomenon has been known for over 30 years and observed for many proteins, however, it is not always possible to observe this DET. Indeed, depending on the redox proteins studied and depending on the electrode material, direct electronic exchange is not always favored. In order to be able to observe the bio electrocatalytic phenomenon, it is then necessary to add a redox molecule (often of low molecular weight) which will act as an electronic transporter. This is mediated electron transfer (TEM) and the molecule used as a "shuttle" is commonly called a redox mediator (Figure 1).

The electron transfer mode (DET or MET) is very often inherent in the biomolecule used and in the electrode material.

There is a very wide variety of redox mediators commonly used in the development of biofuel cells. Among the best known, we find metal complexes (osmium complexes, ferrocene), derivatives of quinones, azines or viologen types.

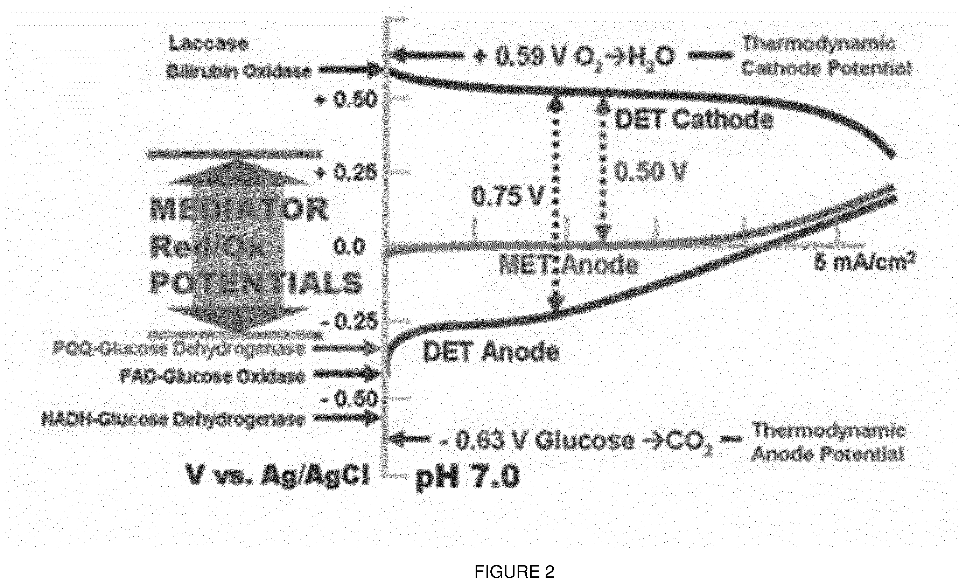

To be a suitable candidate as a redox mediator, the redox probe must therefore meet several criteria :
- The potential of this mediator must thermodynamically favor the Biocatalysis reaction. That is to say that its potential must be higher than that of the enzyme at the anode, and conversely, lower than that of the enzyme at the cathode. (Figure 2).
- Its redox system must be reversible.
- The enzyme / mediator electronic transfer must be efficient to allow good electro-catalysis.
   - The chemical structure of the redox probe must respect a certain size in order to be able to "access" the active site of the enzyme, which is buried in the protein structure in order to be able to perform its role of mediator between the enzyme and the electrode.

The glucose oxidase (GOx) was initially widely used in the field of biotechnology, in particular biosensors. In a few years, it has taken its place in the topic of glucose O₂ biofuel cells. Indeed, it was quickly demonstrated that it is possible to perform an anodic electrode with this enzyme using different electrode materials (carbon nanotubes, nanoparticles, graphene, nanofibers). However, to date it is still difficult to obtain a DET with this enzyme due to its size and its hardly accessible FAD / FADH2 active site.

Given the difficulty of obtaining a DET with GOx without modifying the protein, much work has focused on electron transfer using a redox mediator since the late 1980s. There is a very wide variety of redox mediators commonly used in the elaboration of biofuel cells. Among the best known, we find metal complexes (complexes osmium, ferrocene), derivatives of quinones, azines or viologen types.

The mediation usually takes place with both species (enzyme and mediator) in aqueous solution.

In previous studies, ferrocene and derivatives [4], ferro/ferricyanides [5], and quinone derivatives [6] have found application as redox mediators. Other known mediators include methylene blue, Prussian blue, phenazines, methyl violet, and toluidine blue [7]. However there is usually a problem with such mediators : their insolubility.

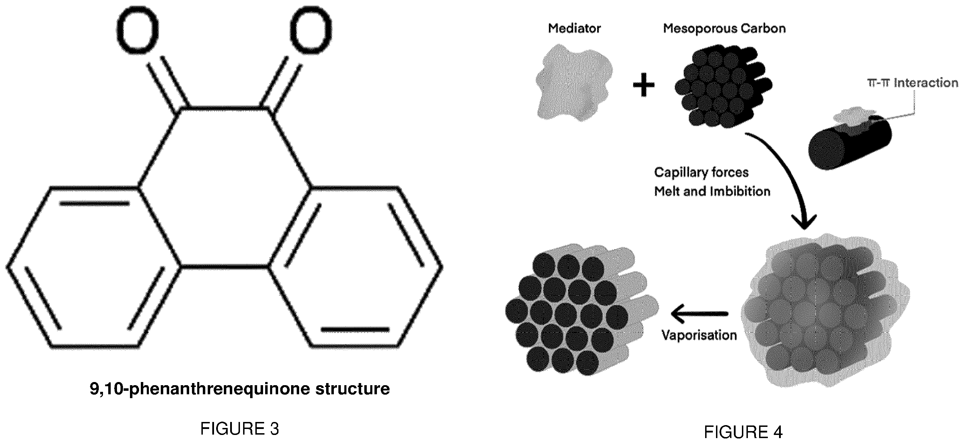

For example, the use of phenanthrenequinone (PQ, Figure 3) as a redox mediator for anodic reaction is limited by a very low solubility of this molecule in water. Even in solution, the mediation signal is lost after a few hours due to the diffusion of the mediator far from the enzyme and/or the electrode surface. The integration of this molecule in a water-based formulation is also problematic to print directly on a paper substrate. On the one hand its insolubility does not allow to obtain a good dispersion and on the other hand, in this configuration, most of the amount of PQ diffuses in the paper when printing and is lost for mediation with the enzyme.

Thus there is still a need to provide a novel redox mediator system, that does not have the prior art disadvantages of the prior art redox mediators due to their insolubility.

### Description of the invention

Therefore the Inventors solved the technical problem by entrapping a redox mediator in a mesoporous carbon structure by a melt diffusion vaporization strategy defined herein [8,9,10] to provide a redox mediator/mesoporous carbon nanocomposite. This allows to increase the enzyme/mediator ratio, to extend the lifetime of bio-anodes thanks to slow release of the mediator, and avoid using organic solvents.

This innovation makes it possible to integrate an insoluble redox mediator into a mesoporous carbon structure to provide a redox mediator/mesoporous carbon nanocomposite. These nanocomposites are then used as electrode materials for the realization of bio-anode seat of glucose oxidation. The realization of this material goes through a heat treatment during which the mediator is brought beyond its melting point (T_{melting}) in the presence of mesoporous carbon. The process can be divided into two phases. First, the redox mediator reaches its melting point, and starts to fill the spaces form by mesopores in the carbon structure. Secondly, a thin layer of redox mediator is created on the available surfaces through a p-p interaction and the excess of mediator is vaporized (Figure 4).

Depending on the size of the mesopores and the specific surface area of the carbon, a greater or lesser amount of mediator can be integrated into the structure. Advantageously, the mediator's redox signal lasts for a longer time compared to formulating it as a solution in immobilization solutions. This afterglow makes it possible to observe a catalytic current over a period greater than the state of the art.

An object of the present invention is therefore a method for preparing a redox mediator/mesoporous carbon nanocomposite, said method comprising the encapsulation of a redox mediator, preferably an insoluble redox mediator (*e.g*. having a solubility in water of about 3.60e-05M) in a mesoporous carbon structure by a melt diffusion vaporization strategy.

For the purposes of the present invention, "mesoporous carbon" refers to a material having pores in the range of 2-50 nm, according to the IUPAC classification of porosity. Traditional porous carbon materials, including activated carbons and carbon molecular sieves, are commonly synthesized using a pyrolysis process with appropriate carbon precursors such as coal, polymers, and carbides, with activation through potassium hydroxide2 or selective etching of metal ions in carbides by halogen gases [11]. The main manufacturers are : Kuraray, BASF, Cabot Norit, Jacobi, Carbons Ingevity Corporation, Dow Corning, Wacker Chemicals, Shin-Etsu, Momentive Performance Materials, Honeywell International, Axens, CECA (Arkema), Zeolyst, Fujian Yuanli Active Carbon, Gelest, ADA-ES, Haycarb, Clariant, CHALCO, Huber.

For the purposes of the present invention, "melt diffusion vaporization strategy" means different weight ratio mixture of a redox mediator (*e.g*. PQ) and mesoporous carbon heated in air (*e.g*. at about 240°C for PQ) for 3 h between the melting point (T_{melting}) and boiling point (T_{boiling}) of the redox mediator (*i.e.* between 200°C and 360°C for PQ). The melted redox mediator was imbibed into the pores by capillary forces. However, only the redox mediator adsorbed on the surface of the carbon pores remained after heating owing to the p-p interaction between the redox mediator and the mesoporous carbon, whereas the redox mediator residue without the p-p interaction evaporated and was eliminated with the air flow because of the high vapor pressure of the redox mediator. Therefore, after cooling, the solidification of redox mediator formed redox mediator-confined in carbons particles with the intimate contact between them.

According to a particular embodiment of the method of the present invention, the method comprises the following steps:
a) mixing a redox mediator with a mesoporous carbon to obtain a mixture ;
b) applying a temperature between the melting and boiling point of the redox mediator to the mixture from step a) ; the excess of redox mediator being vaporized ; and
c) recovering the redox mediator/mesoporous carbon nanocomposite.

The Inventors have unexpectedly observed that during step b) the redox mediator filled the spaces form by mesopores in the carbon structure and formed a thin layer on the available surfaces of the mesoporous carbon through a p-p interaction.

According to a particular embodiment of the method of the present invention, the temperature of step b) can be evaluated as follows: ((T_{boiling}-T_{melting})/4) + T_{melting}.

According to a particular embodiment of the method of the present invention, the redox mediator is an insoluble redox mediator, for example selected from the group consisting of quinones (such as 9,10-phenanthrenequinone (PQ), Naphtoquinone, 1-10 phenanthroline-5,6dione), ferrocene, viologens (such as methyl viologen), azines (such as methylene blue, methylene green, azur A, toluidine blue, and thionine).

According to a particular embodiment of the method of the present invention, the mesoporous carbon is chosen from the group consisting of carbon black (KB600JD, CAS number:1333-86-4), CNovel^{™} products (*e.g*. MJ(4)150, MJ(4)010 and MH) from Toyo Tanso.

Another object of the present invention is also a redox mediator/mesoporous carbon nanocomposite obtainable by a method of the present invention.

Another object of the present invention is the use of a redox mediator/mesoporous carbon nanocomposite of the present invention as an electrode material, in particular a bio-anode.

Such redox mediator/mesoporous carbon nanocomposite can then be used into a fuel cell such as, for example, an enzymatic biofuel cell, an bacterial fuel cell or an organic lithium ion battery or even a sulfur based battery, and said fuel cell into a device such as, for example, an electronic device, in particular for small portable device for applications such as healthcare, environmental monitoring, biodefense, etc...

Another object of the present invention is therefore a biodegradable enzymatic fuel cell comprising a redox mediator/mesoporous carbon nanocomposite of the present invention, and also a device comprising said biodegradable enzymatic fuel cell.

### Brief description of the figures

Figure 1 represents a schematic illustration of a simple arrangement for (A) direct electron transfer (DET) and (B) mediated electron transfer (MET), between the active site of an electrochemically-active enzyme and a solid electrode, during the oxidation of a substrate.
Figure 2 represents the potential windows of redox-mediators.
Figure 3 represents 9,10-phenanthrenequinone structure.
Figure 4 represents thermal treatment.
Figure 5 represents the cyclic voltammograms showing the redox response of (A) fresh electrodes at cycle 2 (B) aged electrodes at cycle 92. (squares) anode with PQ in KB600JD (circles) anode without PQ in KB600JD (triangles) anode without PQ in KB600JD but saturated PQ in the activation liquid.
Figure 6 represents the cyclic voltagramms showing the redox response of electrodes containing different PQ/KB600JD ratios. (crosses) anode with 20% PQ in KB600JD (circles) anode with 70% PQ in KB600JD (squares) anode with 120% PQ in KB600JD.
Figure 7 represents cyclic voltamperometry (A) between 1 and 20 cycles (B) between 20 and 50 cycles (C) after 50 cycles.
Figure 8 represents cyclic voltammograms showing the redox response of electrodes F62D / F64D with Bucky Paper (BP) reference at (A) cycle 2 (B) cycle 20. (circles) F64D (crosses) BP (triangles) F62D

### EXAMPLES

### EXAMPLE 1 : PREPARATION OF A REDOX MEDIATOR / MESOPOROUS CARBON NANOCOMPOSITE

### Protocol vs selected mediator

A 9,10-phenanthrenequinone (PQ) / mesoporous carbon (carbon black (KB600JD)) nanocomposite was prepared by following a melt-diffusion-vaporization strategy. The process temperature was set to be between the melting and the boiling point of the mediator. Experimentally, a two elevated temperature decreased the efficiency and induced a loss of material in a vapor form.

| **PROPERTY OF PQ** | **EXPERIMENTAL AVERAGE** |
|---|---|
| Melting point (°C) | 209 |
| Boiling point°(C) | 360 |
| Water solubility (mol/L) | 3.62e-5 |

First carbon black (KB600JD) and 9,10-phenanthrenequinone (1 g of each for 1:1 ratio) were ground together manually at lab scale or with a 3D mixer for larger amounts, pelletized manually at lab scale or with an hydraulic press for larger amounts, and then heated in an oven, at 240°C, for 3 hours in air. When the 9,10-phenanthrenequinone reached its melting point, it started to fill the spaces form by mesopores in the KB600JD carbon structure. Then, a thin layer of 9,10-phenanthrenequinone was created on the available surfaces (measurement of the specific surface area by BET method (m²/g)) of the KB600JD carbon structure through a p-p interaction, and the excess of mediator was vaporized.

Other redox mediators (*e.g*. ferrocene) can be charged in the same way as long as their degradation temperature is higher than their melting and boiling temperatures.

### Different mesoporous carbon

Four different carbons have been evaluated and loaded with the same ratio of 9,10-phenanthrenequinone (PQ).

A weight ratio mixture of PQ and mesoporous carbon (1:1) was heated in air at 240°C for 3 h between the melting point (200°C) and boiling point (360°C) of PQ. The melted PQ was imbibed into the pores by capillary forces. However, only the PQ adsorbed on the surface of the carbon pores remained after heating owing to the p-p interaction between the PQ and the mesoporous carbon, whereas the PQ residue without the p-p interaction evaporated and was eliminated with the air flow because of the high vapor pressure of the PS. Therefore, after cooling, the solidification of PQ formed PQ-confined in carbons particles with the intimate contact between them.

| Mesoporous carbon references | MJ(4)150-00 | KB600JD | MJ(4)010-00 | MH-00 |
|---|---|---|---|---|
| BET (m²/g) | 270 | 1270 | 1240 | 1417 |
| Mesopores size (nm) | 150 | 8 | 10 | 4 |
| Total pore volume (ml/g) | 0.35 | 1.23 | 2.10 | 1.67 |

### Mass loss vs Mesopore size and Temperature

The same protocole as above described was applied but by weight comparison between initial and post-treatment.

| Mesoporous carbon references | MJ(4)150-00 | KB600JD | MJ(4)010-00 | MH-00 |
|---|---|---|---|---|
| Mass loss at 240°C | 12% | 6% | 5% | 3% |
| Mass loss at 250°C | 33% | NA | NA | NA |
| Mass loss at 300°C | 45% | NA | NA | NA |

Mass loss of 9,10-phenanthrenequinone (PQ) in mesoporous carbons seems to be correlated with the surface area. The less the surface was, the most of the PQ was lost. In the same way, if the temperature was too elevated or far from the melting point, mass loss increased sharply and most of the mediator was lost and escaped from the structure in the form of vapors.

### EXAMPLE 2: RESULTS USING A REDOX MEDIATOR / MESOPOROUS CARBON NANOCOMPOSITE

### Formulation with and without 9,10-phenanthrenequinone (PQ) in mesoporous carbon (black carbon KB600JD)

In order to test the redox mediator/mesoporous carbon nanocomposite in a bio anode configuration, electrodes were fabricated by coating a slurry comprising the nanocomposite directly on a paper substrate.

Slurry was made by mixing the redox mediator/mesoporous carbon nanocomposite or mesoporous carbon alone with a polymeric binder in water. The binder could be carboxymethyl cellulose, polyvinylalcool or polyacrylic acid (water soluble polymers). The slurry contained 3% of redox mediator/mesoporous carbon nanocomposite or 3% of the mesoporous carbon alone, 0.3% of polymer, 1% of Gox enzyme and 95.7% of water. The dispersion was coated on the substrate with a doctor blade in order to get a homogeneous layer that constituted the electrode after drying at 40°C.

The electrochemical characterization was made with a potentiostat in 3 electrodes configuration: Working electrode=bioanode, Counter electrode=platinum wire, and Ag/AgCl reference electrode. The catalytic current was evaluated at 0.2V in each voltammograms.

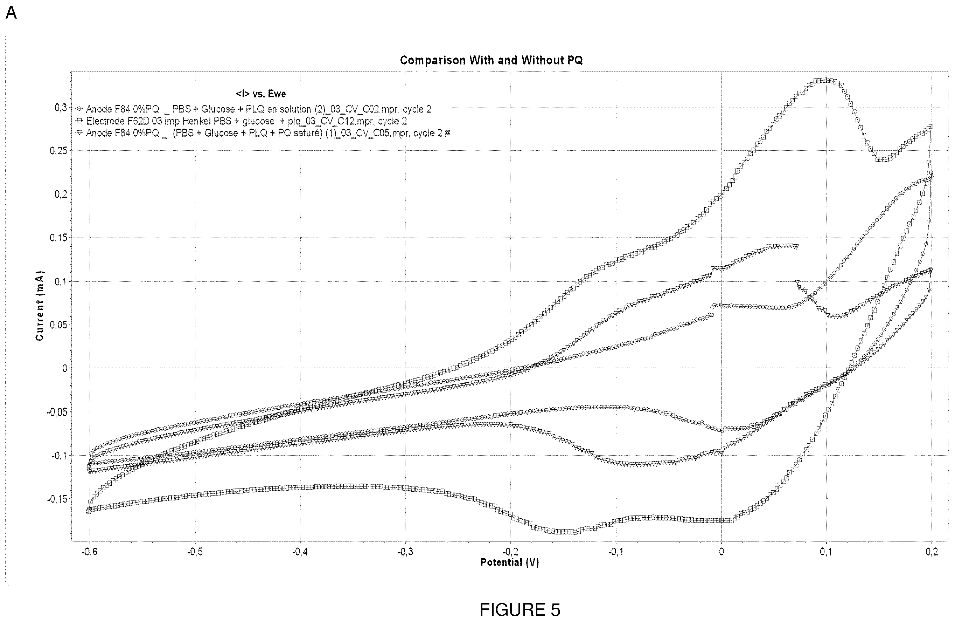

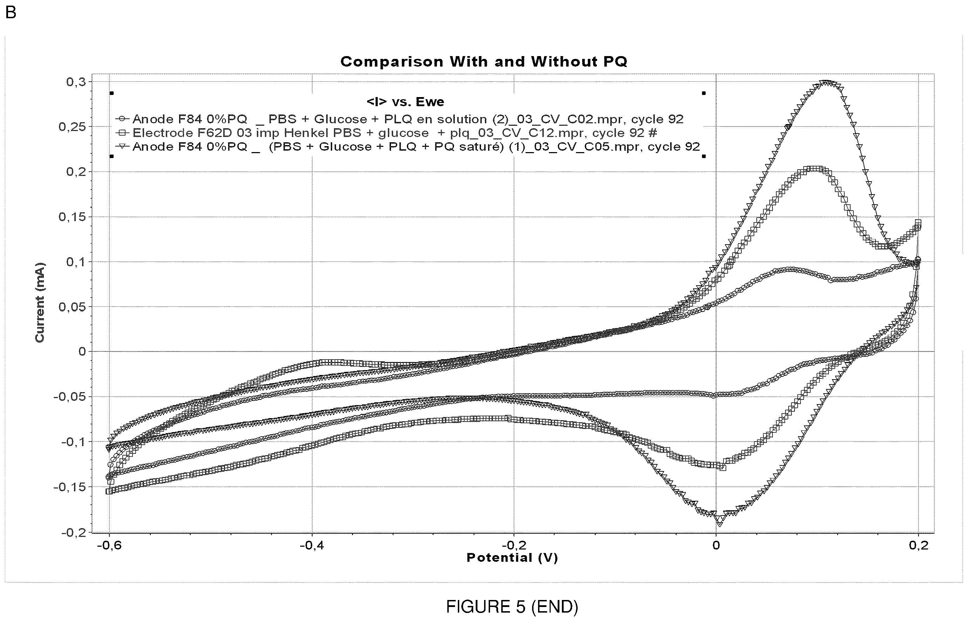

The results were represented in Figures 5A and 5B.

Figure 5A represented the activity of bioanodes with fresh electrodes (at cycle 2). Cyclic voltammograms showed the redox response of electrodes containing PQ or not. Presented cyclic voltammograms were at steady-state operation (scan for 2 mV.s-1). Performance metrics were as follows:
- Circles: Anode with 0% of PQ in KB600JD → Catalytic Peak current at cycle 2 = 0.22mA
- Squares: Anode with PQ in KB600JD →Catalytic Peak current at cycle 2 = 0.27mA
- Triangles: Anode with 0% of PQ in KB600JD but saturated PQ in the activation liquid (3.6*10-5M) → Catalytic Peak current at cycle 2 = 0.11mA.

The results showed that mesoporous containing PQ exhibits a higher catalytic activity than mesoporous without PQ or PQ directly in the activation solution.

Figure 5B represented the activity of bioanodes with aged electrodes (at cycle 92). Cyclic voltammograms showed the redox response of electrodes containing PQ or not. Presented cyclic voltammograms were at steady-state operation (scan for 2 mV.s-1). Performance metrics were as follows:
- Circles: Anode with 0% of PQ in KB600JD. Mediator Peak current at cycle 92 = 0.1 mA.
- Squares: Anode with PQ in KB600JD. Mediator Peak current at cycle 92 = 0.15mA.
- Triangles: Anode with 0% of PQ in KB600JD but saturated PQ in the activation liquid (3.6*10-5M). Mediator Peak current at cycle 92 = 0.10mA.

The results showed that mesoporous containing PQ still exhibited a higher catalytic activity than mesoporous without PQ or PQ directly in the activation solution after 33h operation time.

### Ratio KB/PQ

Several carbon-to-mediator ratios have also been performed to assess the effect of partial/complete filling of the carbon structure.

The same preparations than above were prepared but with different ratio of PQ and KB600JD as follows:

| Formulations | F81A | **F64D** | F82A | **F62D** | F83A |
|---|---|---|---|---|---|
| PQ/carbon KB600JD | 0.20 | 0.50 | 0.70 | 1.00 | 1.20 |

The electrochemical characterization was made as above described.

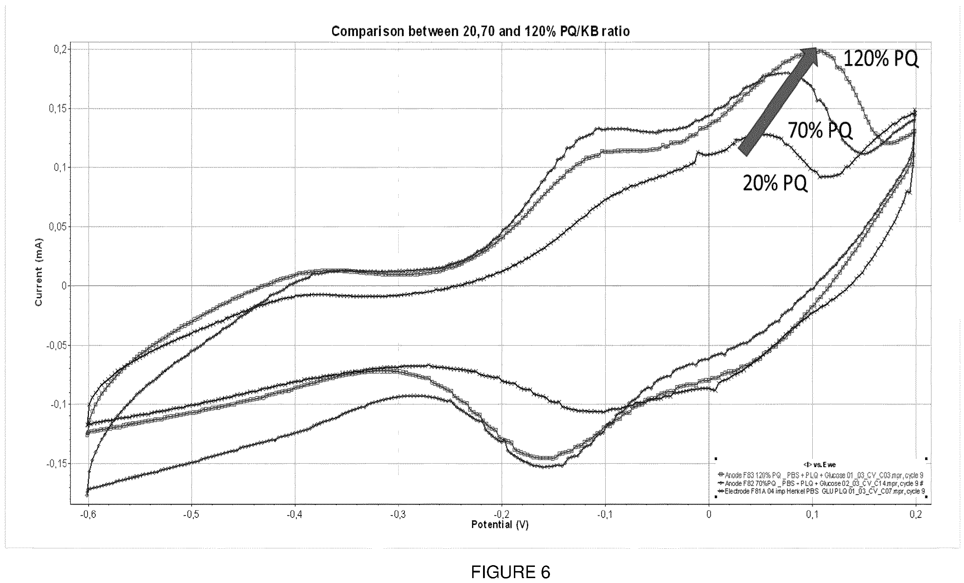

The results were represented in Figure 6.

Figure 6 represented cyclic voltammograms showing the redox response of electrodes containing different PQ/KB ratio. Presented cyclic voltammograms were at steady-state operation (scan for 2 mV.s-1). Performance metrics were as follows:
- Crosses: Anode with 20% of PQ in KB600JD → Mediator Peak current at cycle 9 = 0.12mA
- Circles: Anode with 70% of PQ in KB600JD →Mediator Peak current at cycle 9 = 0.18mA
- Squares: Anode with 120% of PQ in KB600JD → Mediator Peak current at cycle 9 = 0.20mA

As expected, a higher concentration of PQ exhibited a higher current response from the mediator.

### EXAMPLE 3 : REDOX SIGNAL PERSISTANCE OF A REDOX MEDIATOR / MESOPOROUS CARBON NANOCOMPOSITE

### Comparison F64D and F62D

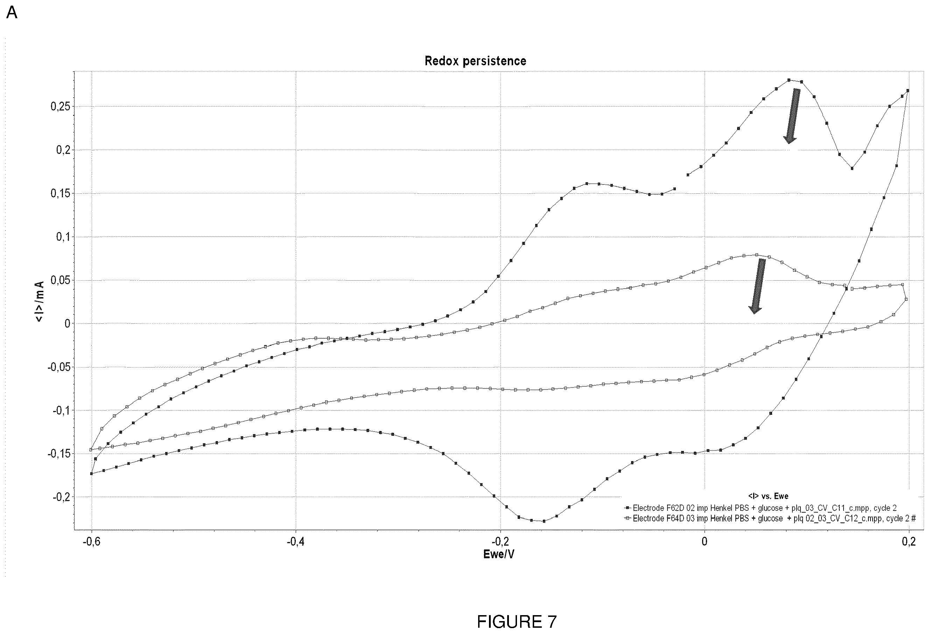

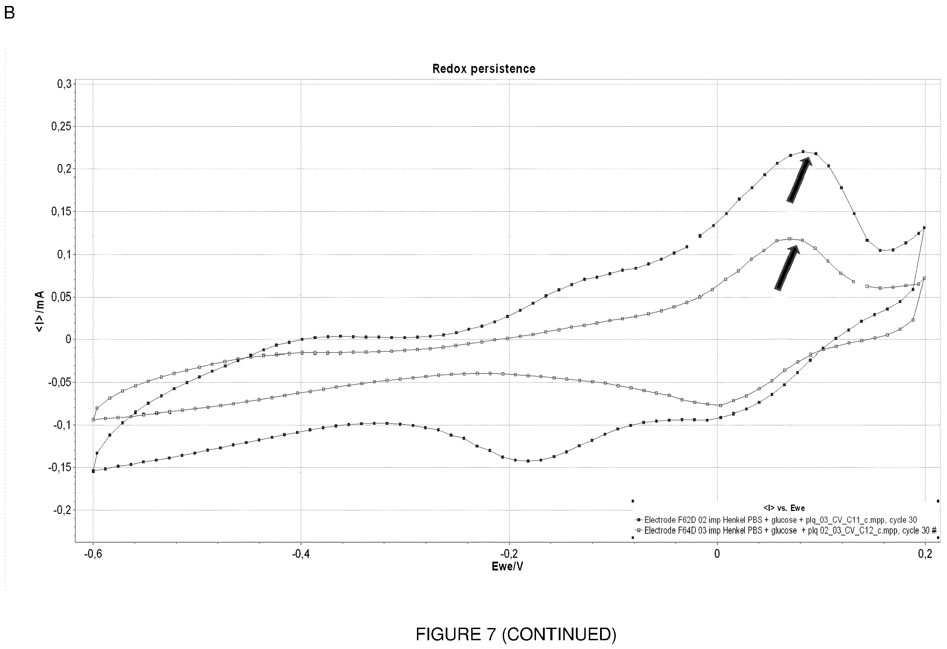

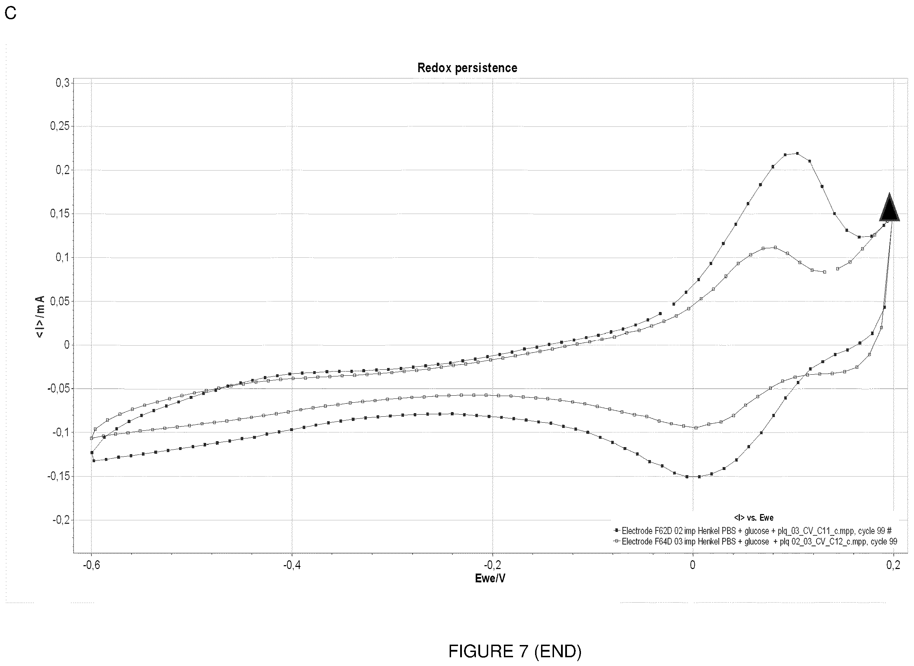

The same protocol as above described was carried out, but in this case, the evolution of both catalytic current and mediator current was followed for both formulations F64D and F62D as above defined.
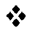 Between cycles 1 and 20: decrease
   The results were represented in Figure 7A.
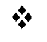 Between cycles 20 and 50: increase
   The results were represented in Figure 7B.
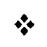 After cycle 50: stabilization
   The results were represented in Figure 7C.

Advantageously, the mediator signal as well as the catalytic current stabilized after a few hours of operation for both mediator concentrations. Moreover, the F62D formulation, which had a KB/PQ ratio of 1, showed a higher catalytic current on the first cycles than the F64D formulation (KB/PQ of 0.66). From 50 cycles, both formulations showed an identical catalytic current.

### Comparison F62D/F64D with BP (Bucky paper)

Bucky paper (BP) electrodes are considered as reference according to the state of the art.

Classically, the mediators were deposited by drop casting directly on the surface of the carbon electrode. After drying, a solution containing the enzymes (glucose oxidase from *Aspergillus niger,* Sigma Aldrich) was also deposited by the same method and dried. The suspension used in said BP reference contained 0.25 mM of PQ and corresponded to a dry deposit of about 4.16 µg/cm².

The enzyme solution used was concentrated to 15.625 mg/ml which corresponded to a dry deposit of about 1.25 mg/cm².

The enzyme to mediator ratio was then 300.

The F62D formulation was composed of 3 % of KB carbon functionalized with PQ (1:1) and 1 % of enzyme. In this case, after deposition, an amount of enzyme of about 1 mg/cm² and an amount of PQ of about 1.5 mg/cm² were obtained.

The enzyme to mediator ratio was then 0.66.

For the F64D formulation, the enzyme to mediator ratio was 1.

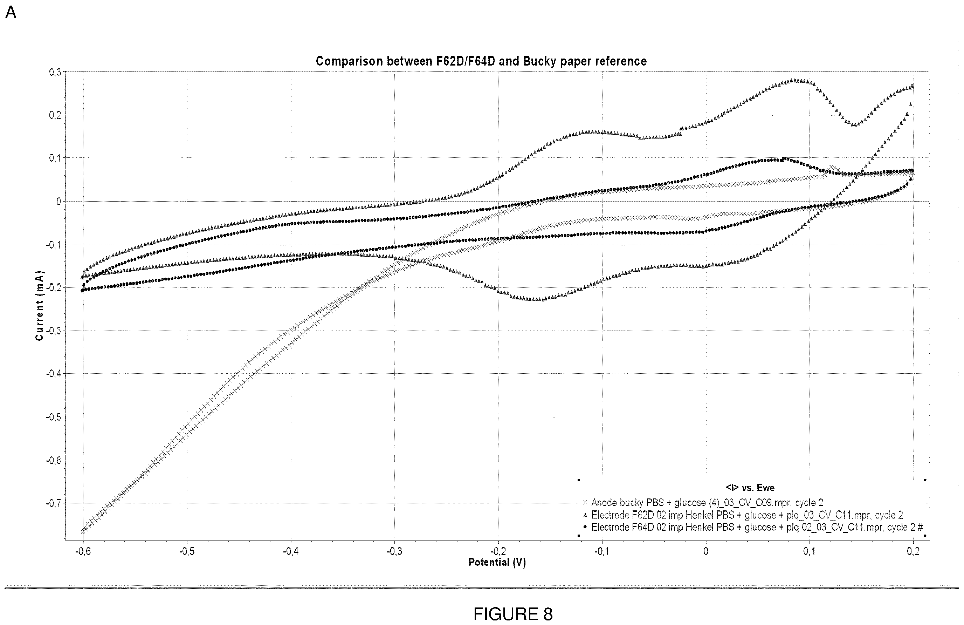

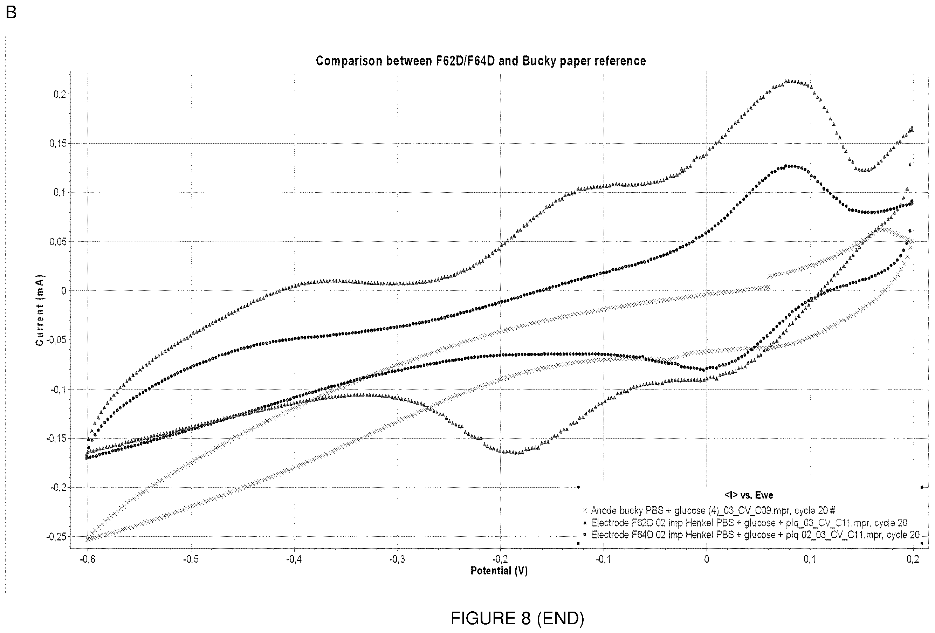

The results were represented in Figures 8A and 8B.

In Figures 8A, functionalized mesoporous carbon with 2 ratios with Bucky paper electrodes at a comparable enzyme loading were compared. Figures 8A showed catalytic response of fresh electrodes. Presented cyclic voltammograms were at steady-state operation (scan for 2 mV.s-1). Performance metrics were as follows:
- F64D (ratio 0.66) : Catalytic current cycle#2 = 0.07mA
- F62D (ratio 1) : Catalytic current cycle#2 = 0.27mA
- Anode Bucky paper (ratio 300) : Catalytic current cycle#2 = 0.07mA

In Figure 8B, we compare functionalized mesoporous carbon with 2 ratios with Bucky paper electrodes at a comparable enzyme loading were compared. Figures 8B showed catalytic response of aged electrodes (7 hours of activity). Performance metrics were as follows:
- F64D (ratio 0.66) : Catalytic current cycle#20 = 0.09mA
- F62D (ratio 1) : Catalytic current cycle#20 = 0.17mA
- Anode Bucky paper (ratio 300) : Catalytic current cycle#20 = 0.05mA

Unlike printed anodes, the electrodes made by drop cast on the bucky paper did not have growth or stabilization phases. The catalytic signal decreased steadily.

### List of references

[1] P. Atanassov, M. Y. EI-Naggar, S. Cosnier and U. Schröder, ChemElectroChem, (2014) 1, 1702-1704
[2] E. Katz and K. MacVittie, Energy Environ. Sci., (2013) 6, 2791
[3] S. Cosnier, A. J. Gross, A. Le Goff and M. Holzinger, J. Power Sources, (2016) 325, 252-263
[4] A. J 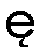 drzak, T. R 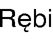 bi's, L. Klapiszewski, J. Zdarta, G. Milczarek, T. Jesionowski, Carbon paste electrode based on functional GOx/silica-lignin system to prepare an amperometric glucose biosensor, Sens. Actuators B Chem. 256 (2018) 176-185, httpS://doi.org/10.1016/j.snb.2017.10.079.
[5] D. Sun, P. Li, Q. Liu, T. Liu, M. Gu, G.-L. Wang, Versatile enzymatic assays by switching on the fluorescence of gold nanoclusters, Anal. Chim. Acta 1095 (2020) 219-225, https://doi.org/10.1016/j.aca.2019.10.035
[6] T. Tamaki, T. Ito, T. Yamaguchi, Immobilization of hydroquinone through a spacer to polymer grafted on carbon black for a high-surface-area biofuel cell electrode, J. Phys. Chem. B 111 (34) (2007) 10312-10319, https://doi.org/10.1021/jp074334n10.1021/jp074334n.s001
[7] G. Rocchitta, A. Spanu, S. Babudieri, G. Latte, G. Madeddu, G. Galleri, S. Nuvoli, P. Bagella, M.I. Demartis, V. Fiore, R. Manetti, P.A. Serra, Enzyme biosensors for biomedical applications: strategies for safeguarding analytical performances in biological fluids, Sensors 16 (2016) 780, https://doi.org/10.3390/s16060780.
[8] C. Tian, J. Wu, Z. Ma, B. Li, X. Zhang, X. Zu, X. Xiang, S. Li, A melt-diffusion strategy for tunable sulfur loading on CC@MoS2 for lithium-sulfur batteries, Energy reports (2020) 6;supl.7,172-180
[9] M-S. Kwon, A. Choi, Y. Park, J.y. Cheon, H. Kang, Y.N. Jo, Y-J. Kim, S.Y. Hong, S.H. Joo, C. Yang, K.T. Lee, Synthesis of ordered mesoporous phenanthrenequinone-carbon via p-p interaction-dependent vapor pressure for rechargeable batteries, Scientific reports (2014) 10;4:7404, doi: 10.1038/srep07404
[10] Patent 10,971,733
[11] A. Eftekhari, Z. Fan, Ordered mesoporous carbon and its applications for electrochemical energy storage and conversion, Mater. Chem. Front. (2017) 1, 1001-1027

## Claims

1. Method for preparing a redox mediator/mesoporous carbon nanocomposite, said method comprising the encapsulation of a redox mediator into a mesoporous carbon structure by melt diffusion vaporization strategy.

2. Method according to claim 1, said method comprising the following steps:
a) mixing a redox mediator with a mesoporous carbon to obtain a mixture ;
b) applying a temperature between the melting and boiling point of the redox mediator to the mixture from step a) ; the excess of redox mediator being vaporized ; and
c)recovering the redox mediator/mesoporous carbon nanocomposite.

3. Method according to claim 2, wherein the temperature of step b) is determined as follows: ((T_{boiling}-T_{melting})/4) + T_{melting}.

4. Method according to any one of claim 1 to 3, wherein the redox mediator is an insoluble redox mediator.
Method according to claim 4, wherein the insoluble redox mediator is chosen from the group consisting of quinones, ferrocen, viologens, and azines.

5. Method according to any one of claims 1 to 5, wherein the mesoporous carbon is carbon black (KB600JD, CAS number:1333-86-4).

6. Redox mediator/mesoporous carbon nanocomposite obtainable par a method according to any one of claims 1 to 6.

7. Use of a redox mediator/mesoporous carbon nanocomposite as defined in claim 7, as an electrode material.

8. Use according to claim 8, wherein the electrode material is a bio-anode.

9. Biodegradable enzymatic fuel cell comprising a redox mediator/mesoporous carbon nanocomposite as defined in claim 7.

10. Device comprising a biodegradable enzymatic fuel cell as defined in claim 10.
